# EUROPEAN PATENT SPECIFICATION

(11) **EP 1 198 249 B1**
(45) Date of publication and mention of the grant of the patent: **19.10.2005**
(21) Application number: 00943919.1
(22) Date of filing: 28.06.2000
(51) Int. Cl.: A61K 39/39, A61K 39/21, A61P 33/06

(54) **USE OF CPG AS AN ADJUVANT FOR HIV VACCINE**
VERWENDUNG VON CPG ALS ADJUVANS FÜR HIVIMPSTOFF
UTILISATION DE CPG COMME ADJUVANT DE VACCIN CONTRE LE VIH

(30) Priority: 29.06.1999 GB 9915205; 31.01.2000 GB 0002200
(43) Date of publication of application: 24.04.2002
(73) Proprietor: Glaxosmithkline Biologicals S.A., 1330 Rixensart (BE)
(72) Inventor: GARCON, Nathalie, B-1330 Rixensart (BE); VOSS, Gerald, B-1330 Rixensart (BE)
(74) Representative: Tyrrell, Arthur William Russell
(86) International application number: PCT/EP2000/005998
(87) International publication number: WO 2001/000232

(56) References cited:
- WO-A-00/23105
- WO-A-00/62800
- WO-A-00/67787
- WO-A-92/06113
- WO-A-96/02555
- WO-A-98/37919
- WO-A-99/16884
- WO-A-99/27961
- WO-A-99/33488
- DEML LUDWIG ET AL: "Immunostimulatory CpG motifs trigger a T helper-1 immune response to human immunodeficiency virus type-1 (HIV-1) gp160 envelope proteins." CLINICAL CHEMISTRY AND LABORATORY MEDICINE, vol. 37, no. 3, March 1999 (1999-03), pages 199-204, XP000982148 ISSN: 1434-6621

## Description

The present invention relates to new vaccine formulations and their use in the treatment or prophylaxis of HIV infections. In particular the invention relates to the use of HIV antigen in conjunction with a CpG oligonucleotide.

HIV is the primary cause of AIDS (acquired immune deficiency syndrome) and is regarded, as one of the worlds most pressing health issues. Although there has been extensive efforts to produce a successful vaccine, efforts to-date have failed to produce one. Accordingly, there remains a need for an improved HIV immunogenic composition.

International Patent Application No. 92/06113 relates to vaccine formulation comprising the envelope protein gp160 and its naturally occurring derivative gp120 adjuvanted 3 deacylated monophosphoryl lipid A and a suitable carrier such as aluminium hydroxide.

International Patent Application No. 99/16884 describes vaccine formulations based on non-envelope HIV proteins, Nef and Tat, particularly fusions of the Nef protein with Tat, and fusions of the Nef protein with Tat or an immunological fusion partner.

Immunomodulatory oligonucleotides contain unmethylated CpG dinucleotides ("CpG") and are known (WO 96/02555, EP 468520). CpG is an abbreviation for cytosine-guanosine dinucleotide motifs present in DNA. Historically, it was observed that the DNA fraction of BCG could exert an anti-tumour effect. In further studies, synthetic oligonucleotides derived from BCG gene sequences were shown to be capable of inducing immunostimulatory effects (both in vitro and in vivo). The authors of these studies concluded that certain palindromic sequences, including a central CG motif, carried this activity. The central role of the CG motif in immunostimulation was later elucidated in a publication by Krieg, Nature 374, p546 1995. Detailed analysis has shown that the CG motif has to be in a certain sequence context, and that such sequences are common in bacterial DNA but are rare in vertebrate DNA.

It is currently believed that this evolutionary difference allows the vertebrate immune system to detect the presence of bacterial DNA (as occurring during an infection) leading consequently to the stimulation of the immune system. The immunostimulatory sequence as defined by Krieg is:

Purine Purine CG pyrimidine pyrimidine and where the CG motif is not methylated. In certain combinations of the six nucleotides a palindromic sequence is present. Several of these motifs, either as repeats of one motif or a combination of different motifs, can be present in the same oligonucleotide. The presence of one or more of these immunostimulatory sequence containing oligonucleotides can activate various immune subsets, including natural killer cells (which produce interferon γ and have cytolytic activity) and macrophages (Wooldrige et al Vol 89 (no. 8), 1977).
Although other unmethylated CpG containing sequences not having this consensus sequence have now been shown to be immunomodulatory.

The present invention provides an improved vaccine formulation comprising a CpG oligonucleotide and an HIV antigen selected from the group HIV gp160, HIV gp120; an HIV Nef protein or derivative thereof linked to either (i) a fusion partner or (ii) an HIV Tat protein or derivative thereof; or an HIV Tat protein or derivative thereof linked to either (i) a fusion protein or (ii) an HIV Nef protein or derivative thereof; or an HIV Nef protein or derivative thereof linked to an HIV Tat protein or derivative thereof and a fusion partner.

By 'fusion partner' is meant any protein sequence that is not Tat or Nef. Preferably the fusion partner is protein D or its lipidated derivative Lipoprotein D, from Haemophilius influenzae B. In particular, it is preferred that the N-terminal third, i.e. approximately the first 100-130 amino acids are utilised. This is represented herein as Lipo D 1/3. In a preferred embodiment of the invention the Nef protein or derivative thereof may be linked to the Tat protein or derivative thereof. Such Nef-Tat fusions may optionally also be linked to a fusion partner, such as protein D.

If present, the fusion partner is normally linked to the N-terminus of the Nef or Tat protein.

Derivatives encompassed within the present invention include molecules with a C terminal Histidine tail, which preferably comprises between 5-10 Histidine residues. Generally, a histidine tail containing n residues is represented herein as His (n). The presence of a histidine (or 'His') tail aids purification. More specifically, the invention provides vaccines comprising proteins with the following structure:

| | | |
|---|---|---|
| Lipo D 1/3 | Nef | His (₆) |
| Lipo D 1/3 | Nef-Tat | His (₆) |
| Prot D 1/3 | Nef | His (₆) |
| Prot D 1/3 | Nef-Tat | His (₆) |
| | Nef-Tat | His (₆) |

In a preferred embodiment the proteins are expressed with a Histidine tail comprising between 5 to 10 and preferably six Histidine residues. These are advantageous in aiding purification. Separate expression, in yeast (Saccharomyces cerevisiae), of Nef (Macreadie IG et al 1993, Yeast 9 (6) 565-573) and Tat (Braddock M et al, 1989, Cell 58 (2) 269-79) has already been reported. Nef protein only is myristilated. The present invention also provides vaccines comprising Nef and/or Tat separately. The DNA and amino acid sequences of representative Nef-His, Tat-His and of Nef-Tat-His fusion proteins as set forth in WO99/16884.

Particularly preferred vaccines according to the invention comprise an HIV fusion protein such as Nef-Tat and optionally an additional HIV protein. Particularly preferred in a vaccine formulation according to the invention is a combination of an HIV fusion protein plus gp120, more particularly Nef-Tat with gp120.

Derivatives encompassed within the present invention also include mutated proteins. The term 'mutated' is used herein to mean a molecule which has undergone deletion, addition or substitution of one or more amino acids using well known techniques for site directed mutagensis or any other conventional method.

Vaccine preparation is generally described in Vaccine Design - The subunit and adjuvant approach (Ed. Powell and Newman) Pharmaceutical Biotechnology Vol. 6 Plenum Press 1995. Encapsulation within liposomes is described by Fullerton, US Patent 4,235,877.

The preferred oligonucleotides preferably contain two or more CpG motifs separated by six or more nucleotides. The oligonucleotides of the present invention are typically deoxynucleotides. In a preferred embodiment the internucleotide in the oligonucleotide is phosphorodithioate, or more preferably a phosphorodithioate bond, although phosphodiester and other internucleotide bonds are within the scope of the invention including oligonucleotides with mixed interaucleotide linkages.

Preferred oligonucleotides have the following sequences:

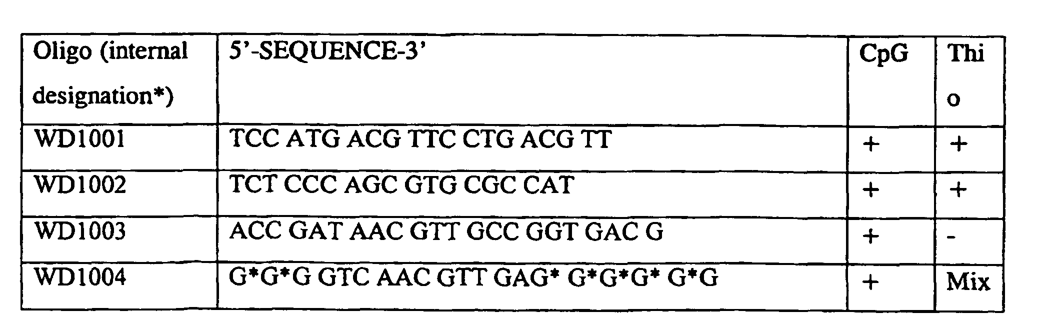

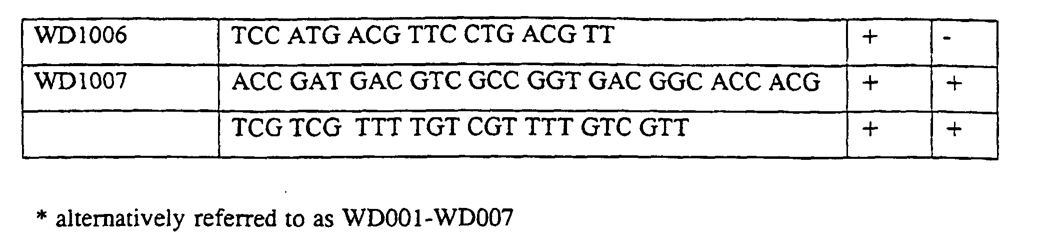

In the above table a + in the Thio column indicates the presence of a thioate modification. 'Mix' indicates a mixture of thioate modification and sequence without thioate modification (the asterisks indicate the linkages with a thioate modification). A - in the Thio column indicates absence of a thioate modification. A + in the CpG column indicates a the presence of a CpG motif and a - in the CpG column indicates absence of a CpG motif. For example WD1005 contains a GpC rather than a CpG motif, thus it is marked with a - in the CpG column of the table. WD1007 contains a palindromic motif (GACGTC) as well as other non-palindromic CpG sequences. This is also within the scope of a CpG oligonucleotide as the term is used in the present application.

The CpG oligonucleotides utilised in the present invention may be synthesized by any method known in the art (eg EP 0 468 520). Conveniently, such oligonucleotides may be synthesized utilising an automated synthesizer. Methods for producing phosphorothioate oligonucleotides or phosphorodithioate are described in US 5,666,153, US 5,278,302 and WO 95/26204.

The amount of protein in each vaccine dose is selected as an amount which induces an immunoprotective response without significant, adverse side effects in typical vaccinees. Such amount will vary depending upon which specific immunogen is employed and how it is presented. Generally, it is expected that each dose will comprise 1-1000 µg of protein, preferably 2-500 µg, most preferably 5-250 µg. An optimal amount for a particular vaccine can be ascertained by standard studies involving observation of appropriate immune responses in subjects. Following an initial vaccination, subjects may receive one or several booster immunisations adequately spaced.

It is also possible to pre-administer the CpG oligonucleotide shortly prior to vaccination with the HIV antigen, for example 1 day before. -

Accordingly, according to another aspect of the invention there is provided a method of treating a human in need thereof either with a vaccine composition comprising an HIV antigen and a CpG oligonucleotide (as hereinabove defined) or with the CpG oligonucleotide followed after a suitable time by the HIV antigen.

There is also provided a kit comprising effective amounts of a CpG oligonucleotide-containing formulation for use as a priming formulation for pre-administration to human patients and an HIV antigen for injection at some suitable time later, as described hereinabove.

Preferred CpG oligonucleotides are those indicated in the table hereinabove.

Suitably the CpG will be present in the range 10 µg per dose to 2000 µg, preferably 50-1000 µg, such as about 50 or about 500 or about 1000 µg per dose.

Suitably the vaccine used in the present invention may comprise a carrier such as an aluminium salt, eg aluminium hydroxide (Al(OH)₃), aluminium phosphate or aluminium phosphate sulfate (alum), or a non-toxic oil in water emulsion or a mixture thereof.

If an aluminium salt (preferably aluminium hydroxide) is used as a carrier it is generally present in the range of 50 to 100 µg, preferably 100 to 500 µg per dose.

Non-toxic oil in water emulsions preferably contain a non-toxic oil, eg squalene and an emulsifier such as (polysorbitan monoleate) Tween 80, in an aqueous carrier such as phosphate buffered saline.

If desired the vaccine used in the present invention may comprise an additional adjuvant, preferably a saponin adjuvant such as QS21 as described for example in WO 9517210, optionally in the presence of a sterol, such as cholesterol as described for example in PCT/EP96/01464. The vaccine of the invention may also comprise monophosphoryl lipid A and derivatives thereof known in the art. A preferred derivative is 3 de-O-acylated monophosphoryl lipid A, described in British Patent No. GB 2 220 211.

Accordingly vaccine formulations of the present invention may additionally comprise other pharmaceutical excipients or immunostimulants. In a preferred embodiment the vaccine formulation additionally comprises an aluminium salt, preferably aluminium hydroxide. In a further embodiment a saponin adjuvant may also be included such as QS21 (Aquila).

The present invention will now be described with reference to the following examples:

### EXAMPLES

### Example 1 - IMMUNOGENICITY STUDIES USING HIV-1 GP120 FORMULATED WITH CPG OR CPG/ALUM

### Evaluation of CpG and CpG/alum in rhesus monkeys

### Experiment outline

An immunogeniciry study was conducted to evaluate the adjuvant effect of CpG in non-human primates. Groups of five monkeys were immunized twice with HIV-1_{W6.1D} gp120 in combination with CpG or CpG/alum. After the second immunization the immune response of the animals was assessed. Antibodies to gp120 and lymphoproliferative as well as cytokine responses were evaluated.

### Groups of monkeys

| **Group** | **antigen** | **Adjuvant** |
|---|---|---|
| 1 | HIV-1_{W6.1} gp120 | CpG/alum |
| 2 | HIV-1_{W6.1} gp120 | CpG |

### Formulation

### Component batches used:

| **COMPONENT** | **BRAND** | **BATCH NUMBER** | **CONCENTRATION (MG/ML)** | **BUFFER** |
|---|---|---|---|---|
| Gp120* | | 47/025 | 0.456 | PBS |
| Al(OH)₃ | Superfos | 96A0089 | 10.380 | H₂O |
| CpG | | WD001 | 5 | H₂O |

| | | | | |
|---|---|---|---|---|
| * gp120 of HIV-1 clone W6.1D (Groenink et al., 1991) | | | | |

### Formulation process:

Formulations were prepared one day before each injection. All incubations were carried out at room temperature with agitation.

### CpG/alum group 1 (500µl/dose)

Gp120 (50µg) was adsorbed on 500µg of Al(OH)₃ for 1 hour. The formulation was buffered with a 10-fold concentrated PO₄/NaCl pH 6.8 solution before addition of 500µg of WD001. After 15 min, 50 µg/ml of thiomersal was added as preservative.

### CpG group 2 (500µl/dose)

Gp120 (50µg) was diluted in PO₄/NaCl buffer pH 6.8 before addition of 500µg of CpG WD001. After 15 min, 50 µg/ml of thiomersal was added as preservative.

### Immunological methods

Five rhesus monkeys *(Macaca mulatta*). per group were immunized twice intramuscularly with 500 µl of vaccine at a four-week-interval. Sera and peripheral blood mononuclear cells (PBMC) were taken at several occasions.

Gp 120-specific antibodies in sera of immunized monkeys were determined in an ELISA as described in Mooij et al., 1998. Briefly, ELISA plates were coated with 1 µg/ml gp120, saturated with BSA and subjected to incubation with rhesus monkey sera followed by α-human biotinylated antibody. Finally, HRP-streptavidin was added and quantified in a color reaction using OPD as substrate.

Lymphoproliferation was assessed by using density gradient-purified PBMC from immunized rhesus monkeys. Cells were seeded in quadruplicates at 1x10⁵ in 100 µl RPMI/5 % FCS per well in round bottom 96 well plates. Then another 100 µl of medium alone or containing soluble gp120 (10 µg/ml) were added and parallel cultures were incubated for 48 hrs. Thereafter, 100 µl culture supernatant were replaced by fresh medium containing 1 µCi [³H]-thymidine. After 16 hrs cells were harvested onto filter plates and incorporated radioactivity was determined in a β-counter. Results are expressed in cpm and in stimulation indices (SI, = cpm antigen-containing cultures/cpm medium alone cultures), SI greater than 3 are considered as a positive response.

Flat bottom 96 well plates were prepared by coating an IFN-γ-specific capture antibody in 50 µl PBS for 4 hrs at 37 °C. The plates were washed three times and PBMC were seeded similar to lymphoproliferation assays. After 48 hrs of culture the plates were washed thrice with PBS/0.05 % Tween 20 and 50 µl of biotinylated secondary IFN-γ-specific antibody diluted in PBS/Tween/1 % FCS were added for 2 hrs. The plates were washed again and a gold-conjugated α-biotin antibody was incubated for 1 hr. After additional washings the ELIspots were visualized by using a silver enhancing kit (50 µl per well). The reaction was stopped after approx. 30 min by adding deionized water. Cytokine-secreting cells were enumerated by microscopic examination.

### Results

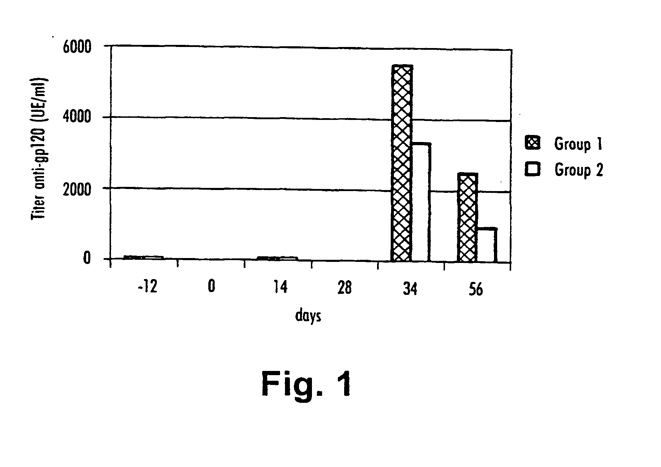

Analysis of gp120-specific antibodies in sera of the monkeys revealed that all animals in the two groups had acquired specific immune responses (Table 1 and Figure 1). Some low responses were detectable in two animals already after one immunization. After the second immunization all animals exhibited high titers of gp120-specific antibodies.

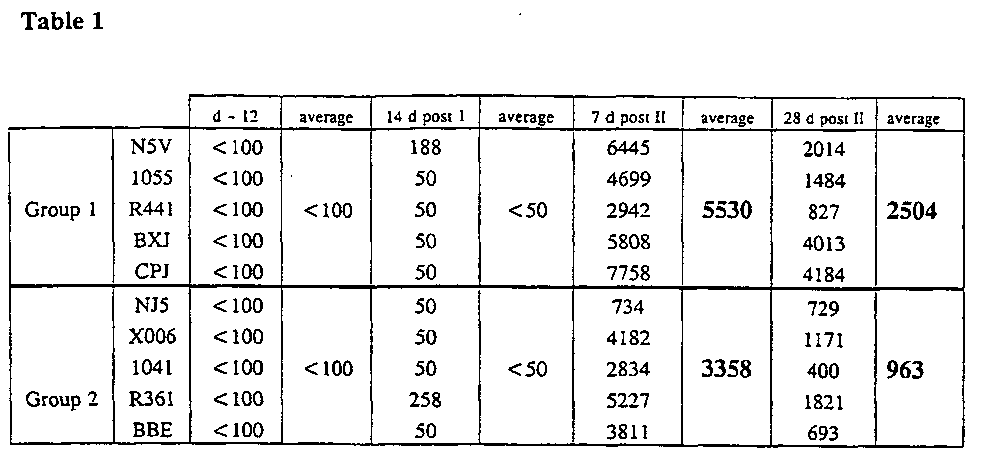

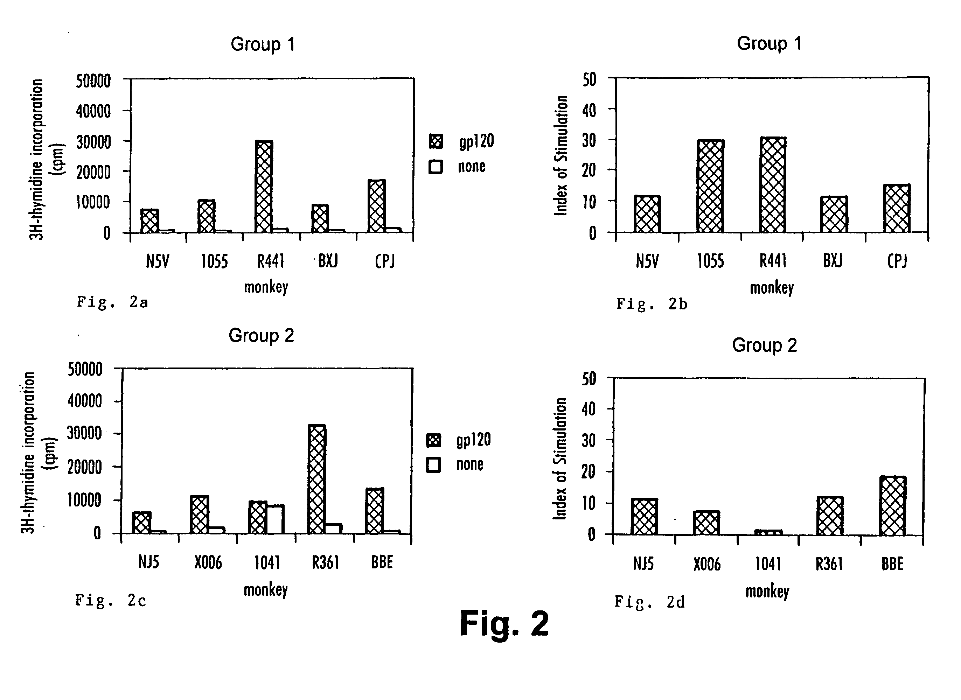

Induction of specific lymphoproliferation by immunization with gp120 in combination with CpG or CpG/alum was evaluated before immunization and 6 days post secondary immunization. All 10 animals did not exhibit any specific lymphoproliferation (SI>3) at the study start (data not shown). In contrast, all animals in group 1 possessed strong lymphoproliferative responses 6 days post boost immunization (Figure 2). Similarly, all except one animal in group 2 showed specific lymphoproliferation.

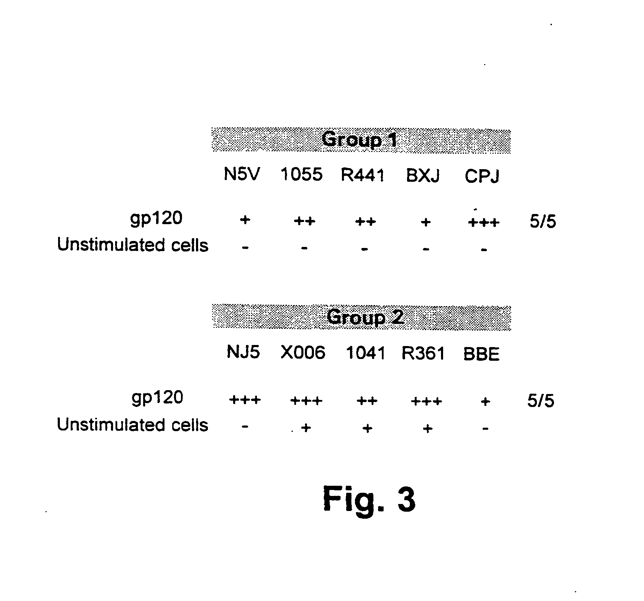

The presence of gp120-specific IFN-γ-secreting cells was investigated in all monkeys before immunization and 6 days after the second dose. IFN-γ-secreting cells could not be evaluated from pre-immunization samples due to technical difficulties. However, such cells were detectable after secondary immunization (Figure 3). All animals in both groups exhibited an antigen-specific response.

### Conclusions

Immunization with HIV-1 gp120 in combination with CpG or CpG/alum induces immune responses in non-human primates. These responses include gp120-specific antibodies, lymphoproliferation and IFN-γ-secreting cells.

### Example 2 - NEF-TAT AND MUTATED NEF-TAT FORMULATION

The antigens prepared according to WO99/16884 were dialysed against NaCl 150mM to eliminate the PO₄ ions that inhibit the adsorption of gp 120 on Al(OH)₃.

### The formulation sequence is as described below:

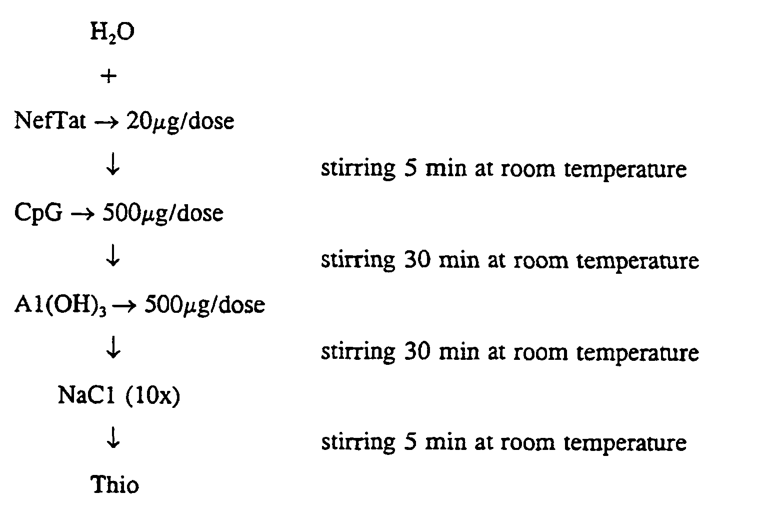

The antigens were incubated with the CpG before adsorption on Al(OH)₃ to favour a potential interaction between the His tail of the Nef-Tat and Nef antigens and the oligonucleotide.

### Formulation Characterisation

The formulation was centrifuged and the supernatant was analsyed by SDS-PAGE (Novex 4-20 % , reducing conditions) to check the adsorption of HIV Nef-Tat. The gel was stained with silver. Gel analysis of the formulation revealed that most of the Nef or Nef-Tat protein was adsorbed onto alum.

### Example 3 - IMMUNIZATION AND SHIV CHALLENGE EXPERIMENT IN RHESUS MONKEYS.

### Vaccine preparation

A vaccine formulation was prepared in accordance with the invention comprising a Nef-Tat fusion protein and gp120 together with an oligonucleotide containing unmethylated CpG dinucleotide motifs and aluminium hydroxide as carrier.

Preparation of CpG oligonucleotide solution: CpG dry powder is dissolved in H₂O to give a solution of 5 mg/ml CpG.

### Preparation of CpG formulation.

The 3 antigens were dialyzed against NaCl 150 mM to eliminate the phosphate ions that inhibit the adsorption of gp120 on aluminium hydroxide.

The antigens diluted in H₂O (100 µg gp120, 20 µg NefTat and 20 µg SIV Nef) were incubated with the CpG solution (500 µg CpG) for 30 min before adsorption on Al(OH)₃ to favor a potential interaction between the His tail of NefTat and Nef antigens and the oligonucleotide (stronger immunostimulatory effect of CpG described when bound to the antigen compared to free CpG). Then Al(OH)₃ (500 µg), 10 fold concentrated NaCl and 1 µg/ml thiomersal as preservative were consecutively added at 5 min intervals.

All incubations were carried out at room temperature with agitation.

### Study

Four rhesus monkeys were immunized intramuscularly at 0, 1 and 3 months with the vaccine composition.

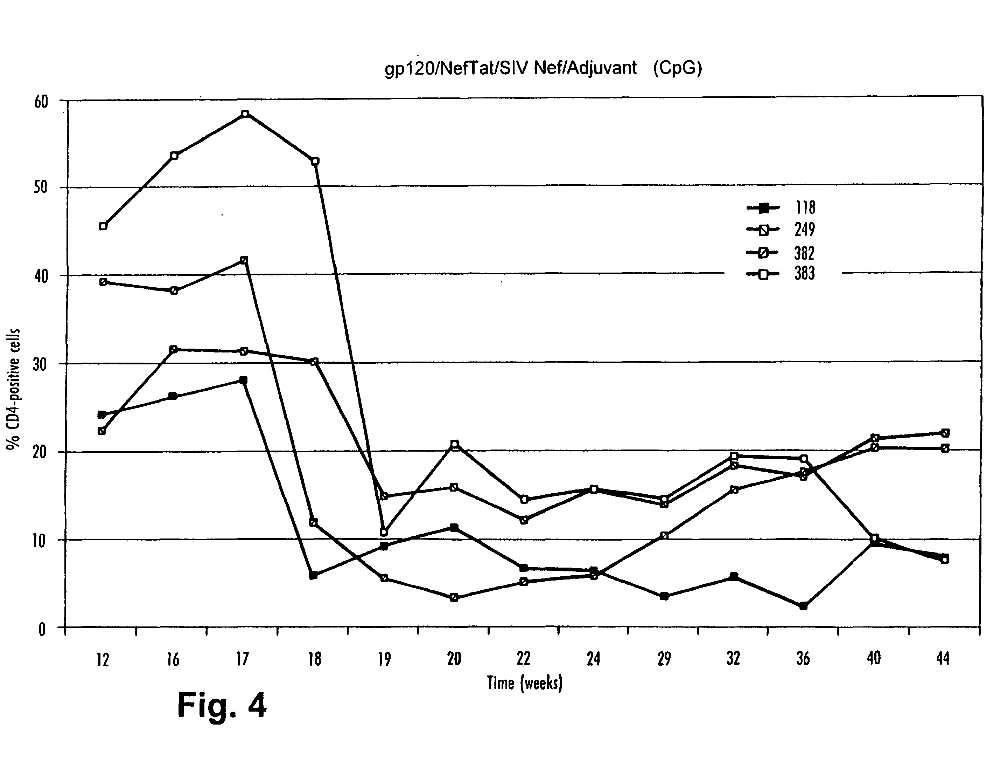

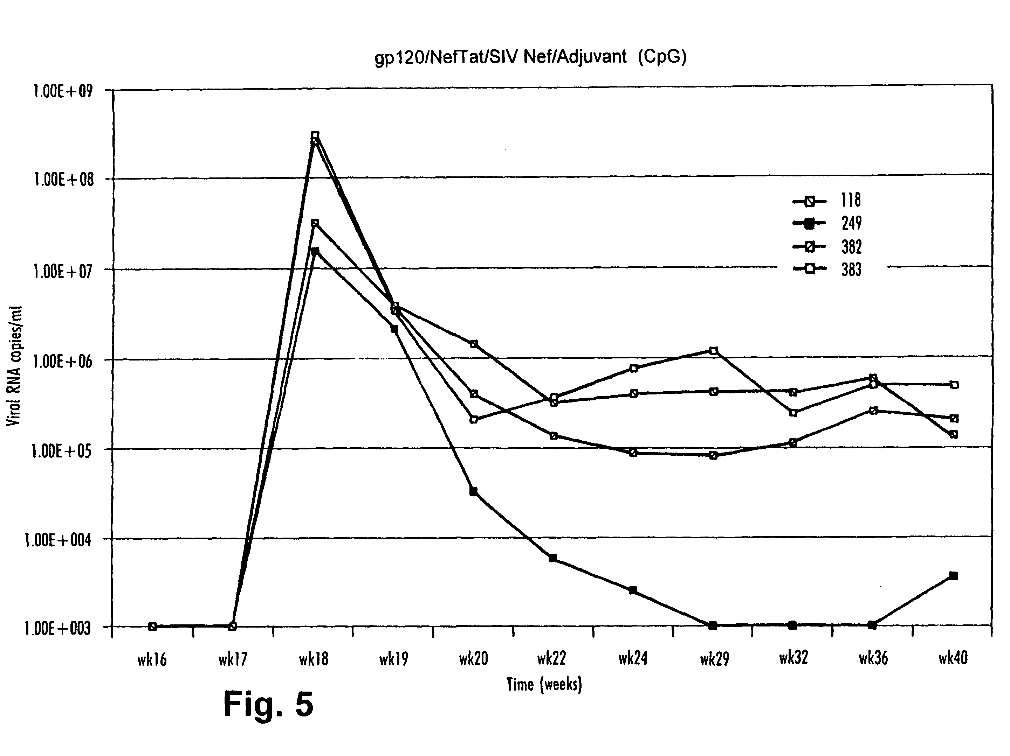

One month after the last immunization all animals were challenged with a pathogenic SHIV (strain 89.6p). From the week of challenge (wk16) blood samples were taken periodically at the indicated time points to determine the % of CD4-positive cells among peripheral blood mononuclear cells by FACS analysis (Figure 4) and the concentration ofRNA viral genomes in the plasma by bDNA assay (Figure 5).

### Results

All animals became infected after challenge with SHIV_{89.6p}.

CD4-positive cells declined after challenge. All four animals exhibited a slight decrease in CD4-positive cells and recovered to baseline levels over time (Figure 4). In contrast, three out of four animals in an adjuvant alone control group exhibited a drop of CD4-positive cells below 5 % and never recovered.

Virus load data are almost the inverse of CD4 data (Figure 5). Virus load decreased in the animals that had received NefTat + gp120 formulated with CpG containing oligonucleotide, but remained high in three out of four control animals which received only an adjuvant.

At week 44 2/4 animals in a control group treated with only an adjuvant had to be euthanized due to AIDS-like symptoms.

### Conclusions

The combination of gp120 and NefTat (in the presence of SIV Nef) with CpG adjuvant prevents the loss of CD4-positive cells and reduces the virus load in animals infected with pathogenic SHIV_{89.6p.}

### Figure legends

Figure 1: HIV-1 gp120-specific antibody responses in immunized rhesus monkeys. Specific antibodies were evaluated in an ELISA. Individual values from multiple time points for each animal are shown in Table 1, and group averages are shown in Table 1 and as a graphic in figure 1.
Figure 2: Gp120-specific lymphoproliferation in immunized rhesus monkeys 6 days post second immunization. PBMC were stimulated with gp120 antigen and lymphoproliferative responses were measured by ³H-thymidine incorporation. Results are expressed in cpm and as SI.
Figure 3: Gp120-specific IFN-γ-secreting cells from immunized rhesus monkeys. IFN-γ-secreting cells were visualized by the ELIspot method. Cytokine-secreting cells resulting in a colored spot were enumerated by microscopic examination and results are expressed semi-quantitatively (- = 0-5, + = 5-15, ++ = 15-35, +++ = 35-50, ++++ = >50).
Figures 4 and 5: CD4 cell count and viral load data from Example 3 in which rhesus monkeys were immunised with Nef-Tat + gp120 formulated with CpG and challenged with pathogenic SHIV.

### References

**Groenink, M., Fouchier, R.A., De Goede, R.E., et al.** (1991). Phenotypic heterogeneity in a panel of infectious molecular human immunodeficiency virus type 1 clones derived from a single individual. *J. Virol.* **65**:1968-1975.

**Mooij, P., Van der Kolk, M., Bogers, M.J.M., et al**. (1998). A clinically relevant HIV-1 subunit vaccine protects rhesus macaques from in vivo passaged simian-human immunodeficiency virus infection. AIDS **12**:F15-F22.

## Claims

1. A vaccine formulation comprising a combination of an HIV fusion protein plus gp120 and an immunostimulatory CpG oligonucleotide.

2. A vaccine as claimed in claim 1 wherein the HIV fusion protein is a Nef-Tat fusion protein.

3. A vaccine as claimed in claim 1 or 2 wherein the Nef-Tat is fused to Protein D, or lipoprotein D or a fragment thereof, from Haemophilius influenzae.

4. A vaccine formulation as claimed in any of claims 1 to 3 additionally comprising an aluminium salt or a saponin adjuvant.

5. A vaccine as claimed in any of claims 1 to 4 wherein the oligonucleotide comprises two CpG dinucleotides.

6. A vaccine as claimed in any of claims 1 to 5 wherein the CpG oligonucleotide is between 15-45 nucleotides in length.

7. A vaccine as claimed in any one of claims 1 to 6 wherein the oligonucleotide is selected from the group:

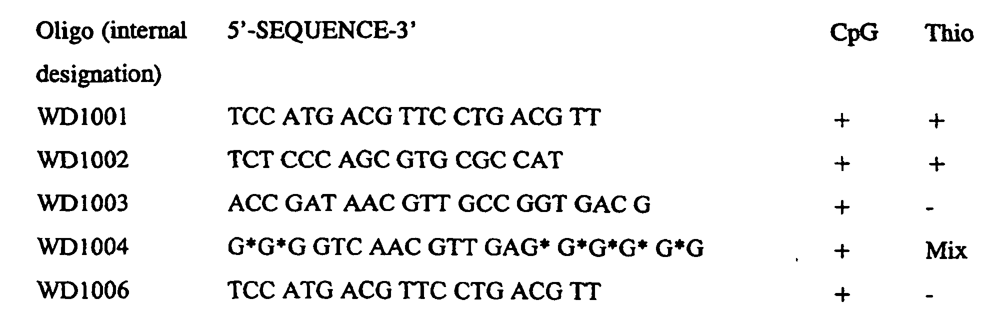

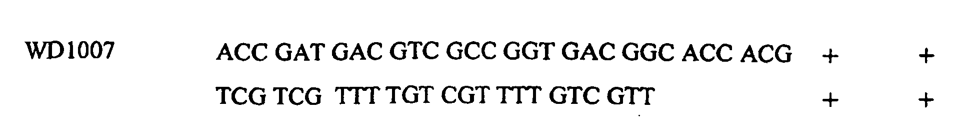

8. A vaccine as claimed in any of claims 1 to 7 wherein the CpG oligonucleotide comprises at least one phosphorothioate internucleotide bond.

9. The use of an HIV fusion protein plus gp120 and an immunostimulatory CpG oligonucleotide in the manufacture of a medicament for the prevention or amelioration of HIV infection in a patient.

10. A vaccine as claimed in any of claims 1 to 9 for use as a medicament.

11. A method of producing a vaccine as claimed in any of claim 1 to 8 comprising admixing an HIV fusion protein plus gp120 and a CpG immunostimulatory oligonucleotide.

12. A kit comprising effective amounts of a CpG oligonucleotide-containing formulation for use as a priming formulation for pre-administration to human patients and an HIV fusion protein plus gp120 for injection at a suitable time later:

## Patentansprüche

1. Impfstofformulierung, die eine Kombination aus einem HIV-Fusionsprotein plus gp120 und ein immunstimulatorisches CpG-Oligonukleotid umfaßt.

2. Impfstoff gemäß Anspruch 1, worin das HIV-Fusionsprotein ein Nef-Tat-Fusionsprotein ist.

3. Impfstoff gemäß Anspruch 1 oder 2, worin das Nef-Tat an Protein D oder Lipoprotein D oder ein Fragment davon aus Hämophilus influenzae fusioniert ist.

4. Impfstofformulierung gemäß einem der Ansprüche 1 bis 3, die zusätzlich ein Aluminiumsalz oder ein Saponin-Adjuvans umfaßt.

5. Impfstoff gemäß einem der Ansprüche 1 bis 4, worin das Oligonukleotid zwei CpG-Dinukleotide umfaßt.

6. Impfstoff gemäß einem der Ansprüche 1 bis 5, worin das CpG-Oligonukleotid zwischen 15 und 45 Nukleotiden lang ist.

7. Impfstoff gemäß einem der Ansprüche 1 bis 6, worin das Oligonukleotid aus der folgenden Gruppe ausgewählt ist:

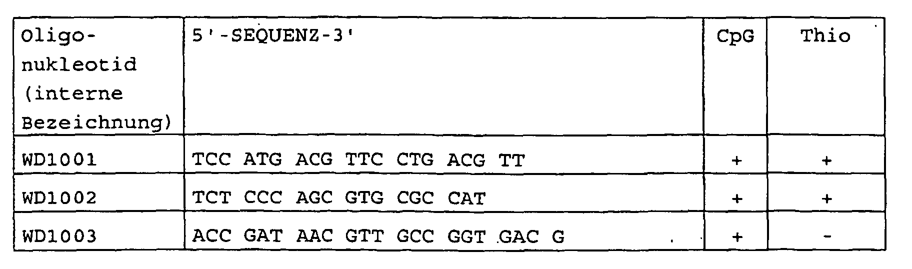

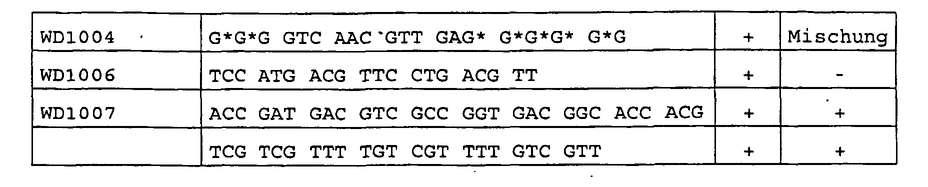

8. Impfstoff gemäß einem der Ansprüche 1 bis 7, worin das CpG-Oligonukleotid wenigstens eine Thiophosphat-Internukleotidbindung umfaßt.

9. Verwendung eines HIV-Fusionsproteins plus gp120 und eines immunstimulatorischen CpG-Oligonukleotids in der Herstellung.eines Medikaments zur Prävention oder Linderung einer HIV-Infektion in einem Patienten.

10. Impfstoff gemäß einem der Ansprüche 1 bis 9 zur Verwendung als Medikament.

11. Verfahren zur Herstellung eines Impfstoffs gemäß einem der Ansprüche 1 bis 8, umfassend das Vermischen eines HIV-Fusionsproteins plus gp120 und eines immunstimulatorischen CpG-Oligonukleotids.

12. Kit, das wirksame Mengen einer CpG-Oligonukleotidhaltigen Formulierungen zur Verwendung als Vorimpfungsformulierung zur Vorverabreichung an menschliche Patienten und eines HIV-Fusionsproteins plus gp120 zur Injektion zu einem geeigneten späteren Zeitpunkt umfaßt.

## Revendications

1. Formulation de vaccin comprenant une association d'une protéine de fusion du VIH plus gp120 et d'un oligonucléotide CpG immunostimulateur.

2. Vaccin selon la revendication 1 dans lequel la protéine de fusion du VIH est une protéine de fusion Nef-Tat.

3. Vaccin selon la revendication 1 ou 2 dans lequel la protéine Nef-Tat est fusionnée à la protéine D, ou à la lipoprotéine D ou à un de ses fragments, d'*Haemophilius influenzae*.

4. Formulation de vaccin selon l'une quelconque des revendications 1 à 3 comprenant en outre un sel d'aluminium ou un adjuvant saponine.

5. Vaccin selon l'une quelconque des revendications 1 à 4 dans lequel l'oligonucléotide comprend deux dinucléotides CpG.

6. Vaccin selon l'une quelconque des revendications 1 à 5 dans lequel l'oligonucléotide CpG a une longueur d'entre 15 et 45 nucléotides.

7. Vaccin selon l'une quelconque des revendications 1 à 6 dans lequel l'oligonucléotide est choisi dans le groupe :

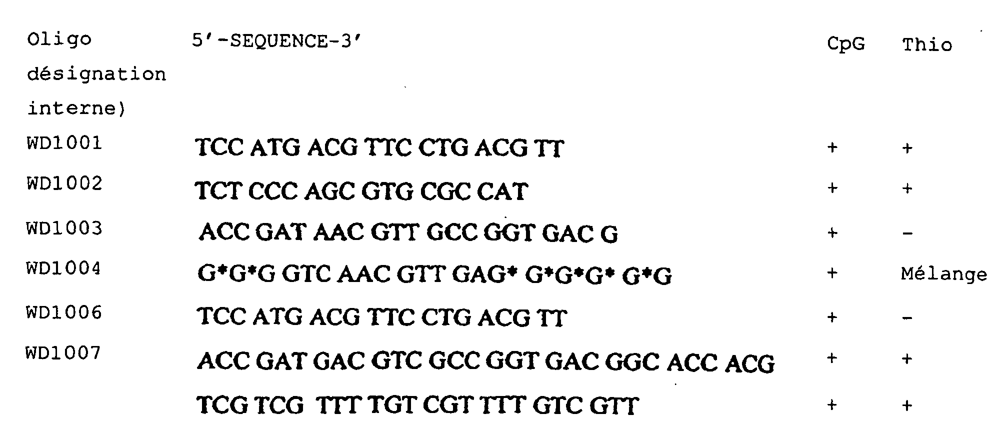

8. Vaccin selon l'une quelconque des revendications 1 à 7 dans lequel l'oligonucléotide CpG comprend au moins une liaison inter-nucléotidique phosphorothioate.

9. Utilisation d'une protéine de fusion du VIH plus gp120 et d'un oligonucléotide CpG immunostimulateur pour fabriquer un médicament pour la prévention ou l'amélioration d'une infection par le VIH chez un patient.

10. Vaccin selon l'une quelconque des revendications 1 à 9 destiné à être utilisé comme un médicament.

11. Procédé pour produire un vaccin selon l'une quelconque des revendications 1 à 8 comprenant le mélange d'une protéine de fusion du VIH plus gp120 et d'un oligonucléotide immunostimulateur CpG.

12. Kit comprenant des quantités efficaces d'une formulation contenant l'oligonucléotide CpG destiné à être utilisé comme formulation d'amorçage pour une pré-administration à des patients humains et une protéine de fusion du VIH plus gp120 pour injection à un moment ultérieur adapté.
